# EUROPEAN PATENT SPECIFICATION

(11) **EP 3 911 305 B1**
(45) Date of publication and mention of the grant of the patent: **07.06.2023**
(21) Application number: 20702409.2
(22) Date of filing: 13.01.2020
(51) Int. Cl.: A61K 9/20, A61K 9/48, A61K 31/167, A61K 31/395

(54) **A METHOD OF MANUFACTURING A PHARMACEUTICAL COMPOSITION COMPRISING NEFOPAM AND ACETAMINOPHEN, AND THE PHARMACEUTICAL COMPOSITION OBTAINED THEREBY**
VERFAHREN ZUR HERSTELLUNG EINER PHARMAZEUTISCHEN ZUSAMMENSETZUNG MIT NEFOPAM UND ACETAMINOPHEN UND DADURCH ERHALTENE PHARMAZEUTISCHE ZUSAMMENSETZUNG
PROCÉDÉ DE FABRICATION D'UNE COMPOSITION PHARMACEUTIQUE COMPRENANT DU NÉFOPAM ET DE L'ACÉTAMINOPHÈNE ET COMPOSITION PHARMACEUTIQUE AINSI OBTENUE

(30) Priority: 14.01.2019 EP 19151603
(43) Date of publication of application: 24.11.2021
(73) Proprietor: APTYS Pharmaceuticals SAS, 63360 Saint Beauzire (FR)
(72) Inventor: BOUTIGNON, Francois, 63000 Clermont-Ferrand (FR); BERNARD, Wahéna, 63410 Manzat (FR); MASSON-SINTEFF, Claudie, 63200 Mozac (FR)
(74) Representative: Holme Patent A/S
(86) International application number: PCT/EP2020/050660
(87) International publication number: WO 2020/148217

(56) References cited:
- CN-A- 102 266 563
- CN-A- 105 030 765
- TU Y H ET AL: "Nefopam hydrochloride degradation kinetics in solution", JOURNAL OF PHARMACEUTICAL SCIENCES, vol. 79, no. 1, 1 January 1990 (1990-01-01), pages 48-52, XP009514465,

## Description

The present invention relates to a method of manufacturing a pharmaceutical composition comprising the two active ingredients nefopam and acetaminophen, a pharmaceutical composition obtained by said method, and a solid unit dosage form comprising said pharmaceutical composition.

The literature indicates that the control of pain, e.g. postoperative pain, remains a challenge for several reasons, including delayed patient recovery and the risk of developing persistent postoperative pain.

Opioids, non-steroidal anti-inflammatory drugs (NSAIDs) and acetaminophen are widely used for the treatment of moderate to severe pain, and they are today the most commonly used analgesics for the management of chronic, inflammatory and postoperative pain.

These agents consistently produce analgesia, but they have a number of undesirable side effects that limit their clinical usefulness. The side effects of opioids include nausea, vomiting, constipation, respiratory depression, urinary retention, sedation, tolerance and physical dependence, while gastrointestinal troubles are frequently observed with NSAIDs, and liver injury is associated with acetaminophen.

This situation has led to the co-administration of combinations of analgesics that have different mechanisms of action through a strategy called 'multimodal' or `balanced' analgesia.

The mechanism underlying the use of multimodal analgesia is that to use analgesics having a different mode of analgesic action, which allows the dose of the analgesics to be reduced and results in a lowered incidence of side effects. The basic goal of this strategy is a synergistic, or at least, additive, analgesic interaction between the combined drugs. However, there is no consensus regarding what the ideal approach to multimodal analgesia is.

Several different multimodal approaches have been suggested in the literature, most of which include the use of opioids.

However, due to the severe side effects of opioids, it may in some situations be preferred to use non-opioids, and here nefopam has proven to be beneficial (Girard et al., Systematic evaluation of the nefopam-paracetamol combination in rodent models of antinociception; 2011).

Nefopam (5-methyl-1-phenyl-1,3,4,6-tetrahydro-2,5-benzoxazocine) is an analgesic that has been used to treat mild to moderate postoperative pain in different clinical settings. It is a non-opioid and non-steroidal drug, that is chemically distinct and pharmacologically unrelated to any of the presently known analgesics (Heel et al., Nefopam: A review of its pharmacological properties and therapeutic efficacy. Drugs 1980; 19: 249-67).

Furthermore, the use of nefopam is advantageous since it does not have any of the known side-effects normally associated with opioids, that is, it does not bind to opioid receptors, does not cause respiratory depression, has no effect on platelet function and does not induce an anti-inflammatory effect.

Its main mechanisms of analgesic action involve the inhibition of serotonin, norepinephrine and dopamine reuptake, and effects on the glutamatergic pathway via modulations of calcium and sodium channels that leads to decreased activation of postsynaptic glutamatergic receptors, such as N-methyl-D-aspartate (NMDA) receptors, which are involved in the development of hyperalgesia.

However, the short elimination half-life of nefopam (four hours) makes it difficult to maintain analgesic efficacy over the normal dosing period (three times daily). Dose escalation of nefopam brings about an increase in the frequency of adverse drug reactions associated with the analgesic, and adverse effects on pulse and blood pressure have been observed following parenteral delivery of therapeutic doses of nefopam.

Accordingly, nefopam is a good candidate for inclusion in multimodal analgesia. One of the compounds nefopam has been combined with, in order to obtain a multimodal analgesia, is acetaminophen (N-acetyl-p-amino-phenol), also known as paracetamol. Acetaminophen is a widespread antipyretic and analgesic, and is accordingly accepted as an effective treatment for the relief of pain and fever in both adults and children. CN 105030765 A, for instance, discloses analgesic compositions comprising nefopam and acetaminophen, which are, preferably, in the form of infusion solutions.

In preparation and storage of pharmaceutical compositions, it is important to provide the active drugs in a pure form. Moreover, it is desirable to achieve this high purity and stability with as simple a formulation as possible. However, one of the problems with pharmaceutical compositions is that impurities may appear in the compositions, e.g. due to choice of synthetic route, the quality of the starting materials, the reaction conditions, the final purification step, the design of the process equipment etc. The presence of such unwanted chemicals, even in trace amount, may influence the efficacy and safety of the pharmaceutical composition.

One of the main impurity problems in relation to acetaminophen, originates as a consequence of both synthesis and degradation of acetaminophen during storage, where acetaminophen is converted into 4-aminophenol.

Even though acetaminophen when stored at room temperature (about 20°C) under dry conditions is considered to be stable, the compound will at elevated temperatures, e.g. in tropical countries, and in the presence of trace moisture degrade rapidly to 4-aminophenol, which subsequently undergoes additional oxidative changes and be converted into p-benzoquinone and hydroquinone, both of which decompose rapidly at room temperature (Fairbrother, J.E. "Acetominophen". In Analytical Profiles of Drug Substances, Vol. 3. K. Florey, Ed. Academic Press, New York, NY, 1974, pp. 1-110.).

Furthermore, in aqueous solutions the degradation of acetaminophen is both acid and base catalyzed and degrades via first order kinetics to 4-aminophenol (Koshy et al., Stability of aqueous solutions of N-acetylp-aminophenol. J. Pharm. Sci. 50: 113-18 (1961)).

Since 4-aminophenol is reported to have both nephrotoxic and teratogenic effects (Németh et al., Determination of paracetamol and its main impurity 4-aminophenol in analgesic preparations by micellar electrokinetic chromatography, Journal of Pharmaceutical and Biomedical Analysis, 2008, vol. 47 (pg. 746-749)), the quantity of 4-aminophenol in the pharmaceutical composition must be strictly controlled.

In a similar manner, undesirable degradation products (impurities) from nefopam can be found in products containing nefopam, and there is accordingly a demand to provide simple formulations and processes for preparation of pharmaceutical compositions containing acetaminophen and nefopam which have low levels of impurities.

Thus, it is a first aspect according to the present invention to provide a stable pharmaceutical composition comprising nefopam and acetaminophen that may be stored in a humid environment and/or at elevated temperatures without the paracetamol degrading to 4-aminophenol. It is a second aspect according to the present invention to provide a stable pharmaceutical composition comprising nefopam and acetaminophen that has undetectable levels of impurities irrespectively of the storage conditions.

It is a third aspect according to the present invention to provide a new manufacturing process arranged for providing a stable pharmaceutical composition.

It is a fourth aspect according to the present invention to provide a new manufacturing process which is operationally simple, easy to handle and applicable at an industrial scale. These and further aspects are achieved according to the present invention by providing a method of preparing a pharmaceutical composition comprising acetaminophen and nefopam, wherein said method comprises
a. in a first process step, providing a wet granulated powder by mixing acetaminophen, one or more excipients and water;
b. in a second process step, mixing the wet granulated powder with nefopam and a lubricant, and
c. in a third process step, forming the pharmaceutical composition, wherein the first process step further comprises drying the wet granulated powder to a water content of between 2 and 5 wt%.

The inventors have surprisingly discovered, that, if the nefopam is added to the mixture in the second process step, instead of in the first process step, the impurities originating from nefopam are reduced to such an extent that said impurities cannot be detected in the final pharmaceutical composition using a conventional HPLC method.

Furthermore, the inventors have surprisingly found that, by using the above method, the provided pharmaceutical composition will be more stable, such that said composition will comprise fewer impurities than when using the known preparation methods for multimodal analgesia comprising acetaminophen and nefopam.

Thus, the inventors of the present invention have found surprising effects on the stability of the acetaminophen and nefopam in the pharmaceutical composition using the above method.

In the present application, the term "impurities" is defined as "substances in the pharmaceutical composition that are not the active pharmaceutical ingredients (API) themselves, i.e. acetaminophen and nefopam, or the excipients used to manufacture the composition", i.e. impurities are unwanted chemicals that remain within the formulation in small amounts and which may influence quality, safety and efficacy of the composition, thereby potentially causing serious health hazards.

The inventors of the present invention have found that the concentration of impurities in the pharmaceutical composition is well below the thresholds specified by e.g. the European, United States, British and German Pharmacopoeias, e.g. employing a conventional HPLC method, which is set to be 1000 ppm or 0.1% w/w.

Analysis of impurities (including degradants) is done using reverse phase HPLC techniques on the respective samples as is known in the art. Calculations of the amount of impurities is expressed as the integrated area percent of the impurity peak(s) divided by the integrated area percent of all drug-related peaks.

The inventors of the present invention have found that a preferred ratio between nefopam and acetaminophen in the pharmaceutical composition is between 1/10 and 1/30, preferably between 1/15 and 1/20.

The water will function as the granulating fluid thereby providing a wet granulation process, and in an advantageous embodiment, the wet granulated powder is obtained by first mixing the acetaminophen and the one or more excipients, and then adding water. The water mixed into the powders will likely form bonds between the respective powder particles thereby locking the particles together.

According to the present invention, the first process step further comprises drying the wet granulated powder to a water content of between 2 and 5 wt%. Once the solvent/water has been substantially removed by drying and the powders have formed a more densely mass containing small amounts of water, then the granulation mixture may optionally be milled/ mixed, homogenised etc.

In the first process step, a number of excipients suitable for use in a granulation step are added to the acetaminophen. Said excipients are preferably selected from the group consisting of granulating agents, diluents, solvents, glidants, surfactants, preservatives, solubilizers, emulsifiers, plasticizers and the like. The number of excipients that can be included in a formulation is not limited.

Examples of diluents/fillers include, but not limited to, celluloses, cellulose acetate, microcrystalline cellulose, co-processed microcrystalline celluloses (such as various grades of Avicel), silicified microcrystalline cellulose, dextrates, dextrin, dextrose, fructose, glyceryl palmitostearate, hydrogenated vegetable oil, kaolin, lactitol, lactose, maltitol, mannitol, maltodextrin, maltose, pregelatinized starch, sodium chloride, sorbitol, starches, sucrose, glucose, trehalose, erythritol, fructose, calcium sulphate, dibasic calcium phosphate, talc and xylitol or a mixture of one or more of said diluents. However, in a preferred embodiment the diluents/filler is microcrystalline cellulose.

Suitable binders include, but not limited to, celluloses such as microcrystalline cellulose, modified celluloses such as low substituted hydroxypropyl cellulose, hydroxypropyl cellulose, hydroxypropyl methylcellulose, hydroxyethylcellulose, hydroxyethyl methylcellulose, cellulose gum, xanthan gum, sugars (such as sucrose, glucose, amylose, maltodextrin, dextrose and the like), starches such as corn or potato starch, pregelatinized starches, polyvinyl alcohol- polyethylene glycol graft copolymer, copovidone, povidone, carbomers, polycarbophil, polyethylene oxide, polyethylene glycol or a combination of suitable binders. However, in a preferred embodiment the binder is povidone K90 and/or Starch.

Examples of disintegrants include, but not limited to, starches, partially pregelatinized starches, sodium starch glycolate, pregelatinized starch, alginic acid, powdered cellulose, croscarmellose sodium, crospovidone, docusate sodium, guar gum, hydroxypropyl cellulose, low substituted hydroxypropyl cellulose, magnesium aluminum silicate, methylcellulose, sodium alginate or a combination of one or more disintegrants. However, in a preferred embodiment the disintegrant is croscarmellose sodium.

In the second process step, a lubricant is also added. Examples of lubricants include, but not limited to, calcium stearate, zinc stearate, magnesium stearate, aluminium stearate, stearic acids, sodium stearyl fumarate, hydrogenated castor oil, light mineral oil, polyethylene glycol, magnesium lauryl sulfate and the like. However, in a preferred embodiment the lubricant is magnesium stearate.

In a preferred embodiment, step a) of the first process step comprises the consecutive steps of:
a' adding a part of the acetaminophen to a first mixing vessel,
a" adding the one or more excipients to the first mixing vessel,
a‴ adding the remaining acetaminophen to the first mixing vessel, and mixing to a homogenous powder.

The acetaminophen and the one or more excipients are preferably dry components, and by layering said components in the mixing vessel it is ensured that the different components, especially acetaminophen, do not clump together. That is the one or more excipients are also functioning as suspending agent (s), i.e. they aid in providing a homogeneous composition during mixing. Without being bound by theory it is believed that the individual excipients ensure that the acetaminophen particles are separated and accordingly evenly distributed in the provided dry mixture.

The ratio of the acetaminophen to the one or more excipients in the dry mixture is preferably about 2:1 to 5:1, more preferably 2.4:1 to 2.8:1 as the inventors have shown that said ratios provide the best results.

In order to obtain a homogenous mixture for the further process steps in the method according to the invention, it is preferred that the compounds after addition to the first mixing vessel are mixed until the dry mixture is homogenous. The mixing conditions will vary depending on the first mixing vessel and the quantity.

Water is thereafter added to the obtained homogenous powder, thereby providing the wet granulated powder of the first process step.

In order to ensure an even distribution of acetaminophen in the dry mixture, as well as reducing the mixing time required for providing a homogeneous dry mixture, it is preferred that half of the acetaminophen is added to the first mixing vessel in step a' and the remaining in step a‴.

In a similar manner, the second process step may be divided into the following consecutive steps:
b' adding a part of the wet granulated powder obtained in the first process step to a second mixture vessel,
b" adding the lubricant and the nefopam to the second mixing vessel,
b‴ adding the remaining of the wet granulated powder obtained in the first process step to the second mixing vessel, and mixing.

In order to obtain a homogeneous mixture, it is preferred that half of the granulated powder obtained in the first process step is added to the second mixing vessel in step b'.

The present invention also relates to a pharmaceutical composition obtained by the method according to the present invention. Said composition comprises acetaminophen and nefopam, wherein the pharmaceutical composition, after storage at 40°C and 75% relative humidity for 4 weeks, contains no more than about 0.05% total impurities based on area percent of drug related HPLC peaks.

In a preferred embodiment, the content of 4-aminophenol, i.e. the main degradation product of acetaminophen, after storage at 40°C and 75% relative humidity for 4 weeks, is less than 0.001% based on area percent of drug related HPLC peaks. Accordingly, this limit is well below the 50 ppm or 0.005% w/w threshold specified by the European, United States, British and German Pharmacopoeias.

In a similar way, the content of impurities originating from nefopam in the pharmaceutical composition after storage at 40°C and 75% relative humidity for 4 weeks, is less than 0.05% based on area percent of drug related HPLC peaks.

In a preferred embodiment, the ratio between nefopam and acetaminophen in the pharmaceutical composition is between 1/10 and 1/30, preferably between 1/15 and 1/20.

The invention also relates to a solid unit dosage form comprising the pharmaceutical composition according to the invention. Said unit dosage form is preferably a tablet or a capsule arranged for oral administration, but other administration forms are also contemplated within the scope of the present invention.

In a preferred embodiment, the unit dosage form comprises between 5 and 100 mg nefopam, preferably between 10 and 50 mg nefopam and even more preferred between 20 and 40 mg nefopam.

The unit dosage form furthermore comprises between 100 and 1000 mg acetaminophen, preferably between 200 and 750 mg acetaminophen and even more preferred between 300 and 600 mg acetaminophen.

In this respect, the median effective analgesic dose (median value and 95% confidence interval) of nefopam and acetaminophen were 30 mg and 500 mg, respectively. Thus, a preferred oral unit dosage form for management of pain comprises the following components

| Components | (mg) |
|---|---|
| Acetaminophen | 500 |
| Microcrystalline cellulose | ≈146.7 |
| Povidone K90 | ≈33,0 |
| Starch | ≈18,3 |
| Anhydrous citric acid | ≈1,5 |
| Nefopam HCL | 30,0 |
| Magnesium stearate | ≈4,0 |

### EXAMPLES

### EFFECT OF PROCESS STEP ON STABILITY OF NEFOPAM

In order to evaluate if the stability of nefopam was affected by the process step to which it was added to the pharmaceutical composition, the following experiments were performed.

The method of manufacture of the pharmaceutical composition comprises the preparation of two phases: the granulated powder (first process step) which contains most components and an external phase (second process step) which contains one or more lubricant.

Nefopam was added either in the granulated powder (first process step) (not according to the invention) or in the external phase (second process step) (according to the invention).

Two batches using the external phase process and two batches using the internal process were prepared as follows.

In the context of the present invention the term % refers to percentage by weight of the composition.

### Example I (the Batch F192H043 is not according to the invention)

### Batch F192H043 - nefopam added in the first process step

### Step a): First process step

A granulated powder was prepared in a first process step, by adding the following to a planetary mixer: acetaminophen, microcrystalline cellulose (a diluent), Starch and Povidone K90 (binders), Croscarmellose sodium (a disintegrant), and Anhydrous citric acid (a pH adjusting agent).

The components were added to a planetary mixer in the following order: First half of acetaminophen, microcrystalline cellulose, starch, PVP K90, nefopam HCL, cross-carmellose sodium, anhydrous citric acid, and finally the second half of acetaminophen.

The powder, 800 g, was mixed for 10 min at 105 rpm.

The final composition of the initial mixture was the following:

| | |
|---|---|
| Acetaminophen | 68.16% |
| Microcrystalline cellulose | 14.30% |
| Povidone K90 | 4.95% |
| Starch | 4.95% |
| Croscarmellose sodium | 0.35% |
| Anhydrous citric acid | 0.20% |
| Nefopam HCl | 4.09% |

Purified water (235 g) was added into the planetary mixer containing the initial mixture under constant stirring from 80 rmp to 115 rpm for 20 min, then at 115 rpm for 20 min.

The resulting wet granulated powder was spread on 4 plates (200 g/plate), the plates were heated at 50°C until the water content reached 2.7 to 3.5%.

The resulting granulated powder was cooled down for 15 min and granulated using a wet granulator with a 1.25 mm grid with a 40 rpm speed. The final granulated powder was homogenised using a Turbula mixer for 5 min at 51 rpm.

### Second process step

In the second process step, half of the granulated powder was poured in a 1L flask and an external phase comprising Magnesium stearate (lubricant) and Microcrystalline cellulose (filler) was added followed by the second half of the granulated powder.

The composition was then mixed using a Turbula mixer (5 min, 51 rpm) .

The composition of the external phase was as follows

| | |
|---|---|
| Magnesium stearate | 1.00% |
| Microcrystalline cellulose | 2.00% |

The final powder (361g) was then tabletted.

### Batch F194H045 - nefopam added in the second process step

### First process step

A granulated powder was prepared as described for batch F192H043 with the exception that nefopam was not part of the initial mixture, which had the following composition.

| | |
|---|---|
| Acetaminophen | 68.16% |
| Microcrystalline cellulose | 14.30% |
| Povidone K90 | 4.95% |
| Starch | 4.95% |
| Croscarmellose sodium | 0.35% |
| Anhydrous citric acid | 0.20% |

Purified water (255 g) was added into the planetary mixer containing the initial mixture under constant stirring from 80 rmp to 115 rpm for 20 min, then to 115 rpm for 20 min.

The resulting wet powder was spread on 4 plates (200 g/plate), the plates were heated at 50°C until the water content reached 2.7 to 3.5%.

The resulting granulated powder was cooled down for 15 min and granulated using a wet granulator with a 1.25 mm grid with a 40 rpm speed. The final granulated powder was homogenised using a Turbula mixer for 5 min at 51 rpm.

### Second process step.

The second process step was conducted as described for batch F192H043, however the composition of the external phase was:

| | |
|---|---|
| Nefopam HCl | 4.09% |
| Magnesium stearate | 1.00% |
| Microcrystalline cellulose | 2.00% |

### Stability test - comparing batch F192H043 (internal) and F194H045 (external)

The tablets were packaged in glass bottles and then placed in stability chambers at 40°C/75%RH.

Samples were taken after two and four weeks and analysed by HPLC according to the following conditions:
Kinetex C18 100A column; mobile phase, A: KH₂PO₄-phosphoric acid buffer, B: Acetonitrile; gradient, 75% A to 30% A for 10 min;
flow rate 1.5 mL/min; oven temperature 30°C; detection 210 nm; volume of injection 10 µL.

The impurities of nefopam were essayed using external standards. Three unknown impurities were detected at 0.068, 1.12, and 1.17 relative retention time (RR).

The LOD (Limit of Detection) of the method is 0.05% and the LOQ (Limit of Quantitation) was 0.0155%.

The results of stability are presented below:

| | **F192H043** | **F194H045** | **F192H043** | **F194H045** |
|---|---|---|---|---|
| | One week | | Two weeks | |
| RR 0.68 | 0.05 | ND | 0.08 | ND |
| RR 1.12 | 0.13 | ND | 0.16 | 0,07 |
| RR 1.17 | 0.08 | < LOQ | 0.09 | <LOQ |

### Example II (the Batch F193H044 is not according to the invention)

### Batch F193H044 - nefopam added in the first process step

### First process step

A granulated powder was prepared as described for batch F192H043 in example I.

The final composition of the powder was the following:

| | |
|---|---|
| Acetaminophen | 68.85% |
| Microcrystalline cellulose | 14.44% |
| Povidone K90 | 5.00% |
| Starch | 5.00% |
| Croscarmellose sodium | 0.35% |
| Anhydrous citric acid | 0.20% |
| Nefopam HCl | 4.13% |

The remaining steps, e.g. drying, and quantities were the same as disclosed for batch F192H043.

### Second process step

The second process step was conducted as described in example I, The composition of the external phase was:
Microcrystalline cellulose 2.02%

The resulting pharmaceutical composition (357 g) was then tabletted.

### Batch F195H046 - nefopam added in the second process step

### First process step

A granulated powder was prepared as described in example I.

The final composition of the powder was the following:

| | |
|---|---|
| Acetaminophen | 68.85% |
| Microcrystalline cellulose | 14.44% |
| Povidone K90 | 5.00% |
| Starch | 5.00% |
| Croscarmellose sodium | 0.35% |
| Anhydrous citric acid | 0.20% |

The remaining steps, e.g. drying, and quantities were the same as disclosed for batch F194H045.

### Second process step

The second process step was conducted as described in example I, The composition of the external phase was:

| | |
|---|---|
| Nefopam HCl | 4.13% |
| Microcrystalline cellulose | 2.02% |

The final powder (373g) was then tabletted.

### Stability test - comparing Batch F193H044 (internal) and F195H046 (external)

The stability test was conducted as described for example I.

The tablets were packaged in glass bottles and then placed in stability chambers at 40°C/75%RH.

Samples were taken after two and four weeks and analysed by HPLC according to the conditions disclosed in example I.

The impurities of nefopam were essayed using external standards. Three unknown impurities were detected at 0.068, 1.12, and 1.17 relative retention time (RR).

The LOD of the method is 0.05% and the LOQ was 0.0155%.

The results of stability are presented below:

| | **F193H044** | **F195H046** | **F193H044** | **F195H046** |
|---|---|---|---|---|
| | One week | | Two weeks | |
| RR 0.68 | <LOQ | ND | 0.05 | ND |
| RR 1.12 | 0.11 | ND | 0.13 | LOQ |
| RR 1.17 | 0.07 | < LOQ | 0.08 | <LOQ |

### Conclusion

The results are combined below. Addition of nefopam was first carried out in an internal phase, e.g. all the components were mixed together before granulation. This process resulted in degradation and appearance of nefopam impurities during accelerated stability studies (column INT in the table below). In contrast, when nefopam was added in an external phase (second process step), the level of impurities was very low (column EXT) .

| **Batch N°** | **F192H043** | **F194H045** |
|---|---|---|
| phase | INT | EXT |
| Nefopam impurities (1 week) | 40°C | 40°C |
| RR 0.68 | 0.05 | ND |
| RR 1.12 | 0.13 | ND |
| RR 1.17 | 0.08 | < LOQ |
| Nefopam impurities (2 weeks) | | |
| RR 0.68 | 0.08 | ND |
| RR 1.12 | 0.16 | 0,07 |
| RR 1.17 | 0.09 | <LOQ |

| **Batch N°** | **F193H044** | **F195H046** |
|---|---|---|
| phase | INT | EXT |
| Nefopam impurities (1 week) | 40°C | 40°C |
| RR 0.68 | <LOQ | ND |
| RR 1.12 | 0.11 | ND |
| RR 1.17 | 0.07 | < LOQ |
| Nefopam impurities (2 weeks) | | |
| RR 0.68 | 0.05 | ND |
| RR 1.12 | 0.13 | LOQ |
| RR 1.17 | 0.08 | <LOQ |

## Claims

1. A method of preparing a pharmaceutical composition comprising acetaminophen and nefopam, wherein said method comprises
a. in a first process step, providing a wet granulated powder by mixing acetaminophen, one or more excipients and water;
b. in a second process step, mixing the wet granulated powder with nefopam and a lubricant, and
c. in a third process step, forming the pharmaceutical composition,
wherein the first process step further comprises drying the wet granulated powder to a water content of between 2 and 5 wt%.

2. The method according to claim 1, wherein the wet granulated powder is obtained by first mixing the acetaminophen and the one or more excipients, and then adding water.

3. The method according to any of the preceding claims, wherein the ratio between nefopam and acetaminophen in the pharmaceutical composition is between 1/10 and 1/30, preferably between 1/15 and 1/20.

4. The method according to any of the preceding claims, wherein step a) of the first process step comprises the following consecutive steps:
a' adding a part of the acetaminophen to a first mixing vessel,
a'' adding the one or more of the excipients to the first mixing vessel,
a‴ adding the remaining acetaminophen to the first mixing vessel, and mixing.

5. The method according to claim 4, wherein about half of the acetaminophen is added to the first mixing vessel in step a' .

6. The method according to any of the preceding claims, wherein step b) of the second process step is divided into the following consecutive steps:
b' adding a part of the wet granulated powder obtained in in the first process step to a second mixture vessel,
b" adding the lubricant and the nefopam to the second mixing vessel,
b‴ adding the remaining of the wet granulated powder obtained in the first process step to the second mixing vessel, and mixing.

7. The method according to claim 6, wherein about half of the wet granulated powder obtained in the first process step is added to the second mixing vessel in step b'.

8. A pharmaceutical composition obtained by the method according to any of the claims 1 - 7 comprising the active ingredients acetaminophen and nefopam, and wherein the pharmaceutical composition, after storage at 40°C and 75% relative humidity for 4 weeks, contains not more than about 0.05% total impurities based on area percent of drug related HPLC peaks.

9. The pharmaceutical composition according to claim 8, wherein the content of impurities originating from nefopam in the pharmaceutical composition, after storage at 40°C and 75% relative humidity for 4 weeks, is less than 0.05% based on area percent of drug related HPLC peaks.

10. The pharmaceutical composition according to claim 8 or 9, wherein the ratio between nefopam and acetaminophen in the pharmaceutical composition is between 1/10 and 1/30, preferably between 1/15 and 1/20.

11. A solid unit dosage form comprising the pharmaceutical composition according to any of the claims 8-10.

12. The solid unit dosage form according to claim 11 wherein the unit dosage form is selected from the group consisting of a tablet and a capsule.

13. The solid unit dosage form according to claim 11 or 12, wherein the unit dosage form comprises between 5 and 100 mg nefopam, preferably between 10 and 50 mg nefopam and even more preferred between 20 and 40 mg nefopam.

14. The solid unit dosage form according to claim 11, 12 or 13, wherein the unit dosage form comprises between 100 and 1000 mg acetaminophen, preferably between 200 and 750 mg acetaminophen and even more preferred between 300 and 600 mg acetaminophen.

## Patentansprüche

1. Verfahren zur Herstellung einer pharmazeutischen Zusammensetzung, umfassend Acetaminophen und Nefopam, wobei das Verfahren umfasst
a. in einem ersten Verfahrensschritt, Bereitstellen eines feuchten granulierten Pulvers durch Mischen von Acetaminophen, einem oder mehreren Hilfsstoffen und Wasser;
b. in einem zweiten Verfahrensschritt, Mischen des feuchten granulierten Pulvers mit Nefopam und einem Schmiermittel, und
c. in einem dritten Verfahrensschritt, Bilden der pharmazeutischen Zusammensetzung,
wobei der erste Verfahrensschritt ferner das Trocknen des feuchten granulierten Pulvers auf einen Wassergehalt zwischen 2 und 5 Gew.-% umfasst.

2. Verfahren nach Patentanspruch 1, wobei das feuchte granulierte Pulver erhalten wird, indem zuerst das Acetaminophen und der eine oder die mehreren Hilfsstoffe gemischt werden und dann Wasser zugegeben wird.

3. Verfahren nach einem der vorhergehenden Patentansprüche, wobei das Verhältnis zwischen Nefopam und Acetaminophen in der pharmazeutischen Zusammensetzung zwischen 1/10 und 1/30, vorzugsweise zwischen 1/15 und 1/20 liegt.

4. Verfahren nach einem der vorhergehenden Patentansprüche, wobei Schritt a) des ersten Verfahrensschritts die folgenden aufeinanderfolgenden Schritte umfasst:
a' Zugeben eines Teils des Acetaminophens zu einem ersten Mischgefäß,
a" Zugeben des einen oder der mehreren Hilfsstoffe zu dem ersten Mischgefäß,
a‴ Zugeben des verbleibenden Acetaminophens zu dem ersten Mischgefäß und Mischen.

5. Verfahren nach Patentanspruch 4, wobei in Schritt a' etwa die Hälfte des Acetaminophens zu dem ersten Mischgefäß zugegeben wird.

6. Verfahren nach einem der vorhergehenden Patentansprüche, wobei Schritt b) des zweiten Verfahrensschritts in die folgenden aufeinanderfolgenden Schritte unterteilt ist:
b' Zugeben eines Teils des in dem ersten Verfahrensschritt erhaltenen feuchten granulierten Pulvers zu einem zweiten Mischgefäß,
b" Zugeben des Schmiermittels und des Nefopams zu dem zweiten Mischgefäß,
b"' Zugeben des verbleibenden Teils des in dem ersten Verfahrensschritt erhaltenen feuchten granulierten Pulvers zu dem zweiten Mischgefäß,
Mischen.

7. Verfahren nach Patentanspruch 6, wobei in Schritt b' etwa die Hälfte des in dem ersten Verfahrensschritt erhaltenen feuchten granulierten Pulvers zu dem zweiten Mischgefäß zugegeben wird.

8. Pharmazeutische Zusammensetzung, erhalten durch das Verfahren nach einem der Patentansprüche 1-7, umfassend die Wirkstoffe Acetaminophen und Nefopam, und wobei die pharmazeutische Zusammensetzung nach Lagerung bei 40 °C und 75 % relativer Luftfeuchtigkeit für 4 Wochen nicht mehr als etwa 0,05 % Gesamtverunreinigungen, bezogen auf Flächenprozent von arzneimittelbezogenen HPLC-Peaks, enthält.

9. Pharmazeutische Zusammensetzung nach Patentanspruch 8, wobei der Gehalt an Verunreinigungen, die von Nefopam stammen, in der pharmazeutischen Zusammensetzung nach Lagerung bei 40 °C und 75 % relativer Luftfeuchtigkeit für 4 Wochen weniger als 0,05 %, bezogen auf Flächenprozent von arzneimittelbezogenen HPLC-Peaks, beträgt.

10. Pharmazeutische Zusammensetzung nach Patentanspruch 8 oder 9, wobei das Verhältnis zwischen Nefopam und Acetaminophen in der pharmazeutischen Zusammensetzung zwischen 1/10 und 1/30, vorzugsweise zwischen 1/15 und 1/20 liegt.

11. Feste Einheitsdosierungsform, umfassend die pharmazeutische Zusammensetzung nach einem der Patentansprüche 8-10.

12. Feste Einheitsdosierungsform nach Patentanspruch 11, wobei die Einheitsdosierungsform ausgewählt ist aus der Gruppe bestehend aus einer Tablette und einer Kapsel.

13. Feste Einheitsdosierungsform nach Patentanspruch 11 oder 12, wobei die Einheitsdosierungsform zwischen 5 und 100 mg Nefopam, vorzugsweise zwischen 10 und 50 mg Nefopam und noch bevorzugter zwischen 20 und 40 mg Nefopam umfasst.

14. Feste Einheitsdosierungsform nach Patentanspruch 11, 12 oder 13, wobei die Einheitsdosierungsform zwischen 100 und 1000 mg Acetaminophen, vorzugsweise zwischen 200 und 750 mg Acetaminophen und noch bevorzugter zwischen 300 und 600 mg Acetaminophen umfasst.

## Revendications

1. Procédé de préparation d'une composition pharmaceutique qui comprend de l'acétaminophène et du néfopam, dans lequel ledit procédé comprend le fait de :
a. dans une première étape opératoire, procurer une poudre granuleuse humide en mélangeant de l'acétaminophène, un ou plusieurs excipients et de l'eau ;
b. dans une deuxième étape opératoire, mélanger la poudre granuleuse humide avec du néfopam et un lubrifiant ; et
c. dans une troisième étape opératoire, obtenir la composition pharmaceutique ;
dans lequel la première étape opératoire comprend en outre le fait de sécher la poudre granuleuse humide afin d'obtenir une teneur en eau qui se situe entre 2 et 5 pour cent en masse.

2. Procédé selon la revendication 1, dans lequel on obtient la poudre granuleuse humide en mélangeant d'abord l'acétaminophène et lesdits un ou plusieurs excipients et en ajoutant ensuite de l'eau.

3. Procédé selon l'une quelconque des revendications précédentes, dans lequel le rapport entre le néfopam et l'acétaminophène dans la composition pharmaceutique se situe entre 1/10 et 1/30, de préférence entre 1/15 et 1/20.

4. Procédé selon l'une quelconque des revendications précédentes, dans lequel l'étape a) de la première étape opératoire comprend les étapes successives indiquées ci-après, dans lesquelles :
a' on ajoute une partie de l'acétaminophène à un premier récipient de mélange ;
a" on ajoute lesdits un ou plusieurs excipients au premier récipient de mélange ;
a‴ on ajoute l'acétaminophène restant au premier récipient de mélange, et on mélange.

5. Procédé selon la revendication 4, dans lequel on ajoute environ la moitié de l'acétaminophène au premier récipient de mélange à l'étape a'.

6. Procédé selon l'une quelconque des revendications précédentes, dans lequel l'étape b) de la deuxième étape opératoire est subdivisée en étapes successives telles qu'indiquées ci-après, dans lesquelles :
b' on ajoute une partie de la poudre granuleuse humide que l'on a obtenue dans la première étape opératoire à un deuxième récipient de mélange ;
b" on ajoute le lubrifiant et le néfopam au deuxième récipient de mélange ;
b‴ on ajoute le reste de la poudre granuleuse humide que l'on a obtenue dans la première étape opératoire au deuxième récipient de mélange, et on mélange.

7. Procédé selon la revendication 6, dans lequel on ajoute au deuxième récipient de mélange, à l'étape b', environ la moitié de la poudre granuleuse humide que l'on a obtenue dans la première étape opératoire.

8. Composition pharmaceutique que l'on obtient par l'intermédiaire du procédé selon l'une quelconque des revendications 1 à 7, qui comprend les ingrédients actifs à savoir l'acétaminophène et le néfopam ; et dans laquelle la composition pharmaceutique, après entreposage à 40 °C et sous une humidité relative de 75 % pendant 4 semaines, ne contient pas plus d'environ 0,05 % d'impuretés au total en se basant sur l'aire en pour cent des pics HPLC liés au médicament.

9. Composition pharmaceutique selon la revendication 8, dans laquelle la teneur en impuretés qui émanent du néfopam dans la composition pharmaceutique après entreposage à 40 °C et sous une humidité relative de 75 % pendant 4 semaines est inférieure à 0,05 % en se basant sur l'aire en pour cent des pics HPLC liés au médicament.

10. Composition pharmaceutique selon la revendication 8 ou 9, dans laquelle le rapport entre le néfopam et l'acétaminophène dans la composition pharmaceutique se situe entre 1/10 et 1/30, de préférence entre 1/15 et 1/20.

11. Forme posologique unitaire solide qui comprend la composition pharmaceutique selon l'une quelconque des revendications 8 à 10.

12. Forme posologique unitaire solide selon la revendication 11, dans laquelle la forme posologique unitaire est choisie parmi le groupe constitué par un comprimé et une capsule.

13. Forme posologique unitaire solide selon la revendication 11 ou 12, dans laquelle la forme posologique unitaire comprend entre 5 et 100 mg de néfopam, de préférence entre 10 et 50 mg de néfopam et de manière encore plus préférée entre 20 et 40 mg de néfopam.

14. Forme posologique unitaire solide selon la revendication 11, 12 ou 13, dans laquelle la forme posologique unitaire comprend entre 100 et 1000 mg d'acétaminophène, de préférence entre 200 et 750 mg d'acétaminophène, et de manière encore plus préférée entre 300 et 600 mg d'acétaminophène.
